# EUROPÄISCHE PATENTSCHRIFT

(11) **EP 0 320 750 B1**
(45) Veröffentlichungstag und Bekanntmachung des Hinweises auf die Patenterteilung: **03.02.1993**
(21) Anmeldenummer: 88120322.8
(22) Anmeldetag: 06.12.1988
(51) Int. Cl.: C07D 231/44, A01N 43/56

(54) **Verfahren zur Herstellung von 4-substituierten 3-Methyl-1-aryl-5-amino-pyrazolen**
Process for the preparation of 4-substituted-3-methyl-1-aryl-5-amino-pyrazoles
Procédé de préparation de méthyl-3-aryl-1-amino-5-pyrazoles substitués en position

(30) Priorität: 16.12.1987 DE 3742612
(43) Veröffentlichungstag der Anmeldung: 21.06.1989
(73) Patentinhaber: BAYER AG, 51368 Leverkusen (DE)
(72) Erfinder: Fürstenwerth, Hauke, Dr., D-5090 Leverkusen 3 (DE)

(56) Entgegenhaltungen:
- EP-A- 0 201 852
- EP-A- 0 233 341
- EP-A- 0 260 521

## Beschreibung

Die Erfindung betrifft ein neues Verfahren zur Herstellung von bekannten 4-substituierten 3-Methyl-1-aryl-5-amino-pyrazolen, welche insektizide Eigenschaften besitzen.

Es ist bereits bekannt, daß man 4-substituierte 3-Alkyl-1-aryl-5-amino-pyrazole erhält, wenn man 4-unsubstituierte 3-Alkyl-1-aryl-5-amino-pyrazole mit elektophilen Reagenzien umsetzt (vgl. EP-A-201 852).
Die Herstellung der als Vorprodukte verwendeten 4-unsubstituierten 3-Alkyl-1-aryl-5-amino-pyrazole erfolgt dabei in der Regel aus geeignet substituierten Acrylnitril-Derivaten und entsprechenden Arylhydrazinen über mehrere verschiedene intermediär zu isolierende Zwischenstufen (vgl. EP-A-201 852).

Die Nachteile einer mehrstufigen Reaktionsführung liegen auf der Hand, insbesondere wenn schlecht zugängliche und damit kostenintensive Ausgangsverbindungen - wie in diesem Fall geeignet substituierte Arylhydrazine - schon in der ersten Stufe eingesetzt werden müssen.

Weiterhin ist bekannt, daß man bestimmte 4-substituierte 1-Aryl-5-amino-pyrazole auch erhält, wenn man entsprechende Arylhydrazine unter bestimmten Reaktionsbedingungen direkt mit geeignet substituierten Acrylnitril-Derivaten cyclisiert (vgl. DE-OS 34 02 308).

Dieses Verfahren eignet sich jedoch nur für bestimmte in 4-Stellung einzuführende Substituenten, d.h. ist nicht universell anwendbar. So gelingt es beispielsweise nicht, α-Alkylthio- oder α-Halogenalkylthio-substituierte β-Dimethyl-amino-acrylnitrile mit Arylhydrazinen direkt zu cyclisieren, ohne daß sich die Ausgangs- und/oder Endprodukte bei den erforderlichen hohen Temperaturen teilweise zersetzen, besonders wenn die Reaktivität der beteiligten Arylhydrazine durch mehrere elektronegative Substituenten stark herabgesetzt ist. Die Verwendung von α-Alkylthio- oder α-Halogenalkylthio-β-alkoxy-substituierten Acrylnitrilen scheitert schon an der schlechten Herstellbarkeit dieser Ausgangsverbindungen. Die insbesondere als Insektizide und Herbizide interessanten 4-Alkylthio- und 4-Halogenalkylthio-5-amino-1-aryl-pyrazole mit mehreren in der Regel elektronegativen Substituenten im Arylteil (vgl. DE-OS 34 02 308 bzw. EP-A-201 852) sind daher auf diesem Wege nicht in befriedigender Ausbeute und Reinheit zugänglich.

Es wurde nun gefunden, daß man 4-substituierte 3-Methyl-1-aryl-5-amino-pyrazole der allgemeinen Formel (I), 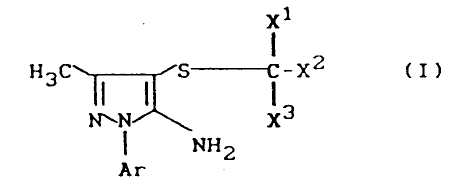
in welcher
- X¹, X² und X³: unabhängig voneinander jeweils für Fluor, Chlor oder Brom stehen und
- Ar: für gegebenenfalls ein- bis fünffach, gleich oder verschieden substituiertes Phenyl steht, wobei als Substituenten jeweils in Frage kommen: Cyano, Nitro, Fluor, Chlor, Brom, Iod, Methyl, Ethyl, n-oder i-Propyl, n-, i-, s- oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest -S(O)ₙ-R³, wobei
- R³: für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht, und
- n: für eine Zahl 0, 1 oder 2 steht, erhält
wenn man 3-Ketobutyraldehyd-dimethylacetal der Formel (II), 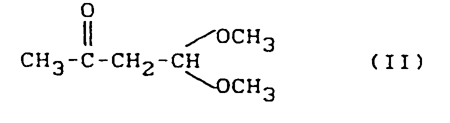
zunächst mit Aminen der Formel (III), 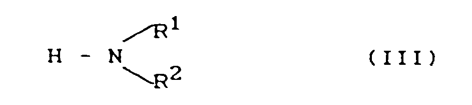
in welcher
- R¹ und R²: unabhängig voneinander jeweils für Wasserstoff, Alkyl, Cycloalkyl oder für jeweils gegebenenfalls substituiertes Aralkyl, Aryl oder Heteroaryl stehen oder gemeinsam mit dem Stickstoffatom, an welches sie gebunden sind, für einen gegebenenfalls substituierten Heterocyclus stehen, der gegebenenfalls weitere Heteroatome enthalten kann,
gegebenenfalls in Gegenwart eines Verdünnungsmittels umsetzt, dann die Reaktionsmischung ohne Isolierung der Zwischenprodukte der Formel (IV), 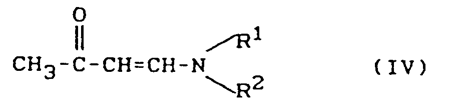
in welcher
- R¹ und R²: die oben angegebene Bedeutung haben,
direkt weiter mit Trihalogenmethansulfenylchloriden der Formel (V), 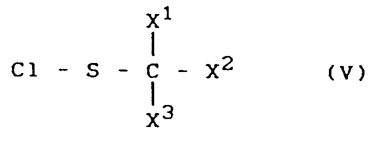
in welcher
- X¹, X² und X³: die oben angegebene Bedeutung haben,
gegebenenfalls in Gegenwart eines Verdünnungsmittels und gegebenenfalls in Gegenwart eines Säurebindemittels umsetzt, dann die Reaktionsmischungen ohne Isolierung der Zwischenprodukte der Formel (VI), 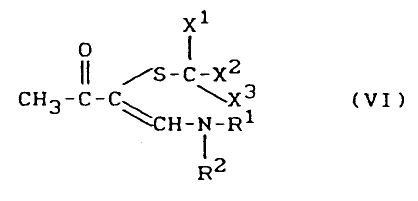
in welcher
- R¹, R², X¹, X² und X³: die oben angegebene Bedeutung haben,
direkt weiter mit Hydroxylaminhydrochlorid in Gegenwart eines Säurebindemittels sowie gegebenenfalls in Gegenwart eines Verdünnungsmittels umsetzt, dann die Reaktionsmischung ohne Isolierung der Zwischenprodukte der Formel (VII), 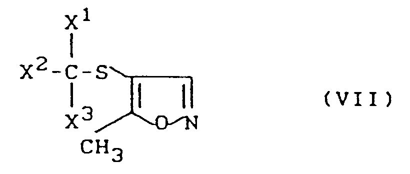
welche unter den gegebenen Reaktionsbedingungen gleich weiterreagieren zu den offenkettigen Tautomeren der Formel (VIIIa), 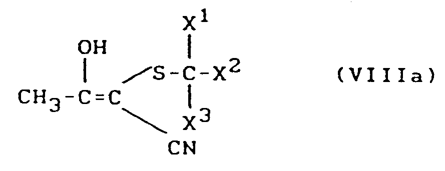
in welcher
- X¹, X² und X³: die oben angegebene Bedeutung haben,
und der Formel (VIIIb), 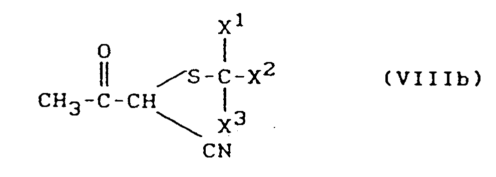
in welcher
- X¹, X² und X³: die oben angegebene Bedeutung haben,
direkt weiter mit Arylhydrazinen der Formel (IX),

Ar - NH - NH₂ (IX)

in welcher
- Ar: die oben angegebene Bedeutung hat,
gegebenenfalls in Gegenwart eines Verdünnungsmittels und in Gegenwart eines sauren Reaktionshilfsmittels umsetzt.

Es ist als ausgesprochen überraschend anzusehen, daß die erfindungsgemäße Umsetzung über 5 Stufen in sogenannten "Eintopfverfahren" bei milden Bedingungen in guter Ausbeute verläuft und Endprodukte in hoher Reinheit liefert, da bei der Polyfunktionalität der verschiedenen eingesetzten Ausgangsprodukte mit einem einheitlichen Reaktionsverlauf über alle Stufen nicht zu rechnen war.

Das erfindungsgemäße Verfahren weist gegenüber dem vorbekannten vielstufigen Herstellungsverfahren eine Reihe von Vorteilen auf. So erhält man die gewünschten Endprodukte der Formel (I) in hohen Ausbeuten in so großer Reinheit, daß in der Regel auf zusätzliche aufwendige Reinigungsoperationen verzichtet werden kann. Als weiterer Vorteil ist zu werten, daß die einstufige Reaktionsführung zu erheblichen Einsparungen von kostenintensiven Ausgangsmaterialien, Hilfsmitteln, Energie- und Abwassermengen führt, was nicht nur unter ökonomischen sondern auch unter ökologischen Aspekten eine deutliche Verbesserung gegenüber dem Stand der Technik bedeutet.

Die mit Hilfe des erfindungsgemäßen Verfahrens herstellbaren 4-substituierten 3-Methyl-1-aryl-5-amino-pyrazole sind durch die Formel (I) allgemein definiert. Bevorzugt herstellbar sind Verbindungen der Formel (I), bei welchen
- X¹, X² und X³: unabhängig voneinander jeweils für Fluor oder Chlor stehen und
- Ar: für gegebenenfalls ein- bis fünffach, gleich oder verschieden substituiertes Phenyl steht, wobei als Substituenten in Frage kommen: Fluor, Chlor, Brom, Methyl, Ethyl, Methoxy, Ethoxy, Trifluormethyl, Trifluormethoxy, Trifluormethylthio, Trifluormethylsulfinyl oder Trifluormethylsulfonyl.

Verwendet man beispielsweise 3-Ketobutyraldehyddimethylacetal, Dimethylamin, Trifluormethansulfenylchlorid und 2,6-Dichlor-4-trifluormethylphenylhydrazin als Ausgangsstoffe, so läßt sich der Reaktionsablauf des erfindungsgemäßen Verfahrens durch das folgende Formelschema darstellen:

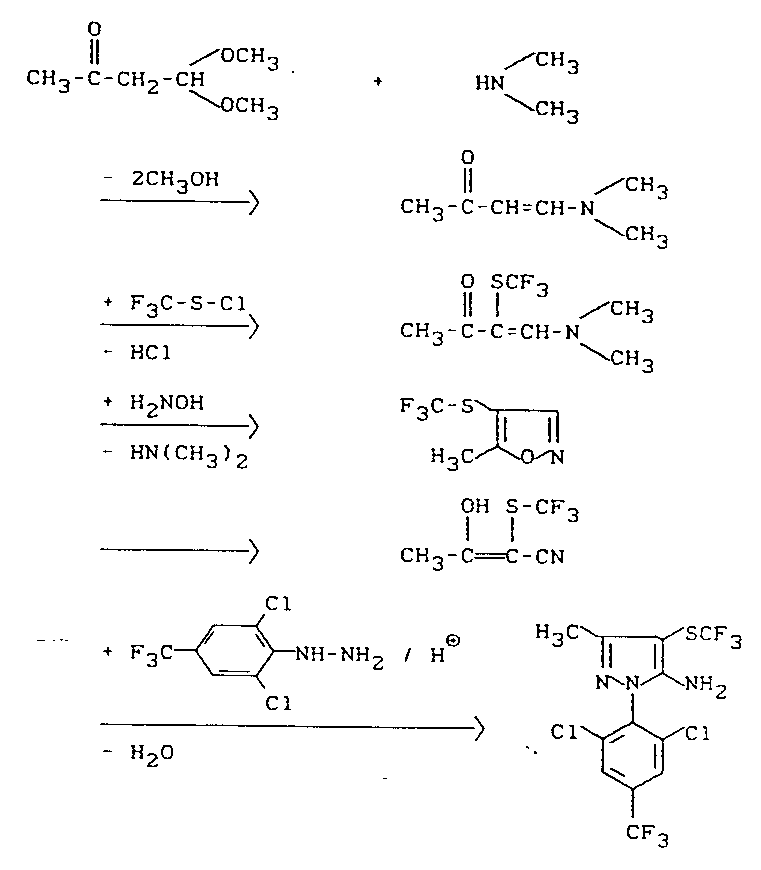

Das zur Durchführung des erfindungsgemäßen Verfahrens als Ausgangsverbindung benötigte 3-Ketobutyraldehyd-dimethylacetal der Formel (II) ist bekannt (vgl. z. B. Chem. Ing. Tech. 46, 395 [1974] oder J. org. Chem. 41, 3765 [1976]).

Die zur Durchführung des erfindungsgemäßen Verfahrens weiterhin als Ausgangsstoffe benötigten Amine sind durch die Formel (III) allgemein definiert. In dieser Formel (III) stehen R¹ und R² unabhängig voneinander vorzugsweise für Wasserstoff, geradkettiges oder verzweigtes Alkyl mit 1 bis 4 Kohlenstoffatomen, für Cycloalkyl mit 3 bis 7 Kohlenstoffatomen oder für jeweils gegebenenfalls ein- bis dreifach gleich oder verschieden substituiertes Phenyl oder Benzyl, wobei als Substituenten in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl oder Ethoxycarbonyl, oder R¹ und R² stehen gemeinsam mit dem Stickstoffatom, an welches sie gebunden sind, für einen fünf- oder sechsgliedrigen gesättigten Heterocyclus, der gegebenenfalls weitere Heteroatome wie insbesondere Stickstoff, Sauerstoff oder Schwefel enthalten kann. R¹ und R² stehen besonders bevorzugt unabhängig voneinander für Wasserstoff, Methyl, Ethyl, n- oder i-Propyl, n-, i-, s- oder t-Butyl, Cyclohexyl, Phenyl oder Benzyl oder gemeinsam mit dem Stickstoffatom, an welches sie gebunden sind, für einen 1-Pyrrolidinylrest, einen 1-Piperidinylrest oder einen 4-Morpholinylrest.

Die Amine der Formel (III) sind allgemein bekannte Verbindungen der organischen Chemie.

Die zur Durchführung des erfindungsgemäßen Verfahrens weiterhin als Ausgangsstoffe benötigten Trihalogenmethansulfenylchloride sind durch die Formel (V) allgemein definiert. In dieser Formel (V) stehen X¹, X² und X³ vorzugsweise für diejenigen Reste, die bereits im Zusammenhang mit der Beschreibung der erfindungsgemäßen Stoffe der Formel (I) als bevorzugt für diese Substituenten genannt wurden.

Die Trihalogenmethansulfenylchloride der Formel (V) sind ebenfalls allgemein bekannte Verbindungen der organischen Chemie.

Die zur Durchführung des erfindungsgemäßen Verfahrens weiterhin als Ausgangsstoffe benötigten Arylhydrazine sind durch die Formel (IX) allgemein definiert. In dieser Formel (IX) steht Ar vorzugsweise für diejenigen Reste, die bereits im Zusammenhang mit der Beschreibung der erfindungsgemäß herstellbaren Stoffe der Formel (I) als bevorzugt für diese Substituenten genannt wurden.

Die Arylhydrazine der Formel (IX) sind bekannt oder erhältlich in Analogie zu bekannten Verfahren (vgl. z. B. EP-A-154 115 oder EP-A-224 831 oder EP-A-187 285 oder EP-A-34 945).

Als Verdünnungsmittel zur Durchführung des erfindungsgemäßen Verfahrens kommen inerte organische Lösungsmittel, oder, falls sie mit Wasser mischbar sind, deren Gemische mit Wasser oder reines Wasser in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder -diethylether; Amide, wie Dimethylformamid, Dimethylacetamid, N-Methylformanilid, N-Methylpyrrolidon oder Hexamethylphosphorsäuretriamid; Ester, wie Essigsäureethylester; Sulfoxide oder Sulfone, wie Dimethylsulfoxid oder Tetramethylensulfon (Sulfolan); Alkohole, wie Methanol, Ethanol, Propanol, Butanol, Ethylenglykol, dessen Monomethyl- oder Monoethylether, deren Gemische mit Wasser oder auch reines Wasser.

Besonders bevorzugt als Verdünnungsmittel sind reines Wasser oder Wasser-Alkohol-Gemische, wie beispielsweise Wasser-Methanol- oder Wasser-Ethanol-Gemische.

Das erfindungsgemäße Verfahren wird bei einigen Reaktionsschritten vorzugsweise in Gegenwart eines geeigneten Säurebindemittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Mit besonderem Vorzug verwendet man schwach basische anorganische Verbindungen wie beispielsweise Natriumhydrogencarbonat oder Natriumacetat.

Das erfindungsgemäße Verfahren wird im letzten Reaktionsschritt vorzugsweise in Gegenwart eines sauren Reaktionshilfsmittels durchgeführt. Als solche können übliche Protonen- oder Lewis-Säuren verwendet werden. Mit besonderem Vorzug verwendet man anorganische Mineralsäuren, wie Salzsäure oder Schwefelsäure; aliphatische oder aromatische Carbon- oder Sulfonsäuren, wie Essigsäure, Methansulfonsäure oder p-Toluolsulfonsäure; Lewis-Säuren, wie Bortrifluorid, Eisentrichlorid, Aluminiumtrichlorid oder Zinkdichlorid oder saure Ionenaustauscher.

Mit besonderem Vorzug verwendet man Salzsäure als Reaktionshilfsmittel im letzten Reaktionsschritt.

Die Reaktionstemperaturen können bei der Durchführung des erfindungsgemäßen Verfahrens in einem größeren Bereich variiert werden. Im allgemeinen arbeitet man bei Temperaturen zwischen -20 °C und +150 °C, vorzugsweise bei Temperaturen zwischen 0 °C und +120 °C.

Zur Durchführung des erfindungsgemäßen Verfahrens setzt man pro Mol an 3-Ketobutyraldehyddimethylacetal der Formel (II) im allgemeinen 1.0 bis 2.0 Mol, vorzugsweise 1.0 bis 1.5 Mol an Amin der Formel (III), 1.0 bis 1,5 Mol, vorzugsweise 1.0 bis 1.2 Mol an Trihalogenmethansulfenylchlorid der Formel (V) und 1.0 bis 1.5 Mol, vorzugsweise 1.0 bis 1.2 Mol an Säurebindemittel, weiterhin 0.7 bis 1.5 Mol, vorzugsweise 0.9 bis 1.2 Mo an Hydroxylaminhydrochlorid sowie eine äquivalente Menge an zusätzlichem Säurebindemittel, 0.7 bis 1,2 Mol, vorzugsweise 0.8 bis 1.0 Mol an Arylhydrazin der Formel (IX) und 1.0 bis 10.0 Mol, vorzugsweise 1.0 bis 5.0 Mol an saurem Reaktionshilfsmittel ein.

In einer bevorzugten Ausführungsform geht man so vor, daß zunächst das 3-Ketobutyraldehyddimethylacetal der Formel (II) mit einer entsprechenden Menge an Amin der Formel (III) in einem geeigneten Verdünnungsmittel, vorzugsweise in Wasser, mehrere Stunden im Temperaturbereich zwischen 10 °C und 30 °C gerührt wird, die so erhaltene Reaktionsmischung wird anschließend mit einer entsprechenden Menge Säurebindemittel, vorzugsweise mit Natriumhydrogencarbonat und danach im Temperaturbereich zwischen -20 °C und +10 °C mit einer entsprechenden Menge von Trihalogenmethansulfenylchlorid versetzt. Diese Reaktionsmischung wird nach kurzer Reaktionszeit weiter verdünnt; vorzugsweise verwendet man an dieser Stelle einen Alkohol wie beispielsweise Ethanol als Verdünnungsmittel, gibt dann eine entsprechende Menge Hydroxylaminhydrochlorid sowie eine äquivalente Menge an Säurebindemittel, vorzugsweise Natriumacetat zu und erhitzt dann für 1 bis 2 Stunden auf Siedetemperatur. Nach Abkühlen der Reaktionsmischung gibt man die erforderliche Menge an saurem Reaktionshilfsmittel, vorzugsweise Salzsäure und Arylhydrazin der Formel (IX) zu und erhitzt wiederum für mehrere Stunden auf Siedetemperatur.

Die Reaktionsdurchführung, Aufarbeitung und Isolierung der Reaktionsprodukte erfolgt nach allgemein üblichen Methoden, beispielsweise durch Entfernen des organischen Verdünnungsmittels, Ausfällen des Reaktionsproduktes in Wasser, Absaugen und Trocknen des so erhältlichen Produktes.

Alternative Ausführungsformen des erfindungsgemäßen Verfahrens bestehen darin, daß man gegebenenfalls auch Zwischenprodukte der Formel (VI) und/oder Zwischenprodukte der Formel (IV) isolieren kann und in getrennter Reaktion weiter umsetzen kann.

Die mit Hilfe des erfindungsgemäßen Verfahrens herstellbaren 4-substituierten 3-Methyl-1-aryl-5-amino-pyrazole der Formel (I) sind bekannte Insektizide (vgl. EP-A-201 852).

### Herstellungsbeispiel

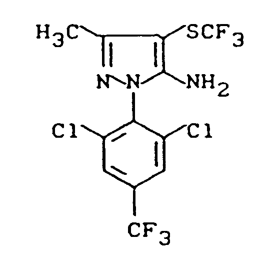

26,5 g (0.2 Mol) 3-Ketobutyraldehyddimethylacetal und 19 g (0.22 Mol) 53 %ige wäßrige Dimethylaminlösung werden 16 Stunden bei Raumtemperatur gerührt, mit 100 ml Wasser verdünnt und mit 18,5 g (0.22 Mol) Natriumhydrogencarbonat versetzt und dann auf 0 °C abgekühlt. Bei 0 °C bis 5 °C leitet man unter Kühlung innerhalb von 30 Minuten 30 g (0.22 Mol) Trifluormethansulfenylchlorid ein und rührt anschließend weitere 30 Minuten ohne Kühlung nach, wobei während 15 Minuten ein leichter Stickstoffstrom durch die Reaktionsmischung geleitet wird. Anschließend gibt man 200 g Ethanol, 13 g (0.18 Mol) Hydroxylaminhydrochlorid und 15 g (0.18 Mol) Natriumacetat zu, erhitzt für 90 Minuten auf Rückflußtemperatur, kühlt dann auf 30 °C bis 40 °C ab, gibt dann 35 g (0.36 Mol) konzentrierte Salzsäure und 42 g (0.17 Mol) 2,6-Dichlor-4-trifluormethylphenylhydrazin zu und erhitzt für weitere 16 Stunden auf Rückflußtemperatur. Zur Aufarbeitung werden 200 g Lösungsmittel abdestilliert, die resultierende Suspension abgekühlt, das ausgefallene Produkt abgesaugt mit 300 ml Wasser gewaschen und im Vakuum bei 50 °C getrocknet.

Man erhält 71 g (91,5 % der Theorie) an 5-Amino-3-methyl-1-(2,6-dichlor-4-trifluor-methylphenyl)-4-trifluormethylthio-pyrazol vom Schmelzpunkt 146 °C -148 °C.

## Patentansprüche

1. Verfahren zur Herstellung von 4-substituierten 3-Methyl-1-aryl-5-amino-pyrazolen der allgemeinen Formel (I) 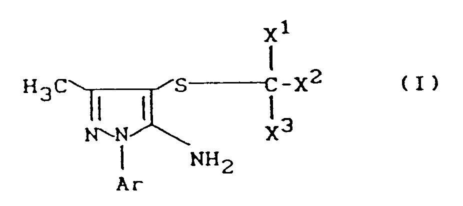 in welcher
X¹, X² und X³ unabhängig voneinander jeweils für Fluor, Chlor oder Brom stehen und
Ar für gegebenenfalls ein- bis fünffach, gleich oder verschieden substituiertes Phenyl steht, wobei als Substituenten jeweils in Frage kommen: Cyano, Nitro, Fluor, Chlor, Brom, Iod, Methyl, Ethyl, n-oder i-Propyl, n-, i-, s- oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest -S(O)ₙ-R³, wobei
R³ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht, und
n für eine Zahl 0, 1 oder 2 steht,
dadurch gekennzeichnet, daß man 3-Ketobutyraldehyddimethylacetal der Formel (II), 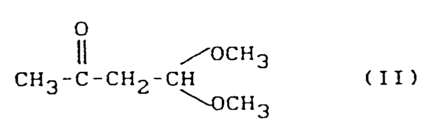 zunächst mit Aminen der Formel (III), 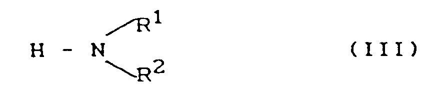 in welcher
R¹ und R² unabhängig voneinander jeweils für Wasserstoff, Alkyl, Cycloalkyl oder für jeweils gegebenenfalls substituiertes Aralkyl, Aryl oder Heteroaryl stehen oder gemeinsam mit dem Stickstoffatom, an welches sie gebunden sind, für einen gegebenenfalls substituierten Heterocyclus stehen, der gegebenenfalls weitere Heteroatome enthalten kann,
gegebenenfalls in Gegenwart eines Verdünnungsmittels umsetzt, dann die Reaktionsmischung ohne Isolierung der Zwischenprodukte der Formel (IV), 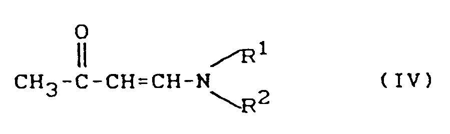 in welcher
R¹ und R² die oben angegebene Bedeutung haben,
direkt weiter mit Trihalogenmethansulfenylchloriden der Formel (V), 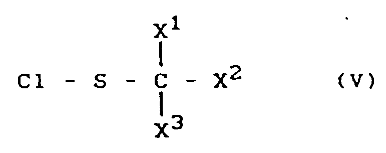 in welcher
X¹, X² und X³ die oben angegebene Bedeutung haben,
gegebenenfalls in Gegenwart eines Verdünnungsmittels und gegebenenfalls in Gegenwart eines Säurebindemittels umsetzt, dann die Reaktionsmischungen ohne Isolierung der Zwischenprodukte der Formel (VI), 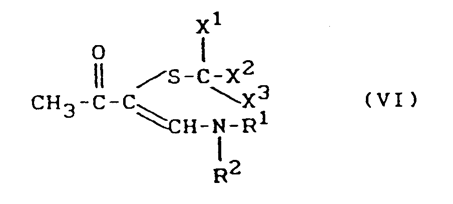 in welcher
R¹, R², X¹, X² und X³ die oben angegebene Bedeutung haben,
direkt weiter mit Hydroxylaminhydrochlorid in Gegenwart eines Säurebindemittels sowie gegebenenfalls in Gegenwart eines Verdünnungsmittels umsetzt, dann die Reaktionsmischung ohne Isolierung der Zwischenprodukte der Formel (VII), 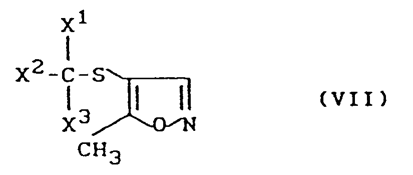 welche unter den gegebenen Reaktionsbedingungen gleich weiterreagieren zu den offenkettigen Tautomeren der Formel (VIIIa), 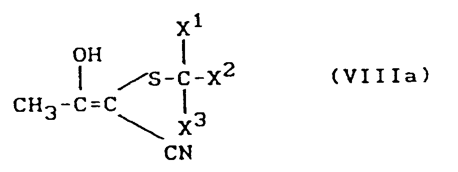 in welcher
X¹, X² und X³ die oben angegebene Bedeutung haben,
und der Formel (VIIIb), 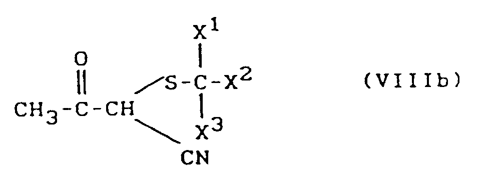 in welcher
X¹, X² und X³ die oben angegebene Bedeutung haben,
direkt weiter mit Arylhydrazinen der Formel (IX),
Ar - NH - NH₂ (IX)
in welcher
Ar die oben angegebene Bedeutung hat,
gegebenenfalls in Gegenwart eines Verdünnungsmittels und in Gegenwart eines sauren Reaktionshilfsmittels umsetzt.

2. Verfahren gemäß Anspruch 1 zur Herstellung von Verbindungen der Formel (I), in welcher
X¹, X² und X³ unabhängig voneinander jeweils für Fluor oder Chlor stehen und
Ar für gegebenenfalls ein- bis fünffach, gleich oder verschieden substituiertes Phenyl steht, wobei als Substituenten in Frage kommen: Fluor, Chlor, Brom, Methyl, Ethyl, Methoxy, Ethoxy, Trifluormethyl, Trifluormethoxy, Trifluormethylthio, Trifluormethylsulfinyl oder Trifluormethylsulfonyl.

3. Verfahren gemäß Anspruch 1 bis 2, dadurch gekennzeichnet, daß man als Verdünnungsmittel inerte organische Verdünnungsmittel oder deren Gemische mit Wasser oder Wasser allein einsetzt.

4. Verfahren gemäß Anspruch 1 bis 3, dadurch gekennzeichnet, daß man als Verdünnungsmittel aliphatische, alicyclische oder aromatische, gegebenenfalls halogenierte Kohlenwasserstoffe, Ether, Amide, Ester, Sulfoxide, Alkohole, deren Gemische mit Wasser oder Wasser einsetzt.

5. Verfahren gemäß Anspruch 1 bis 4, dadurch gekennzeichnet, daß als Verdünnungsmittel Wasser oder Wasser-Alkohol-Gemische eingesetzt werden.

6. Verfahren gemäß Anspruch 1 bis 5, dadurch gekennzeichnet, daß in Gegenwart einer anorganischen oder organischen Base als Säurebindemittel gearbeitet wird.

7. Verfahren gemäß Anspruch 1 bis 5, dadurch gekennzeichnet, daß man im letzten Reaktionsschritt in Gegenwart eines sauren Reaktionshilfsmittels der Reihe anorganische Mineralsäuren, wie Salzsäure oder Schwefelsäure; aliphatische oder aromatische Carbon-oder Sulfonsäuren, wie Essigsäure, Methansulfonsäure oder p-Toluolsulfonsäure; Lewis-Säuren, wie Bortrifluorid, Eisentrichlorid, Aluminiumtrichlorid oder Zinkdichlorid oder saure Ionenaustauscher arbeitet.

8. Verfahren gemäß Anspruch 1 bis 7, dadurch gekennzeichnet, daß die Umsetzung bei Temperaturen zwischen 0°C und +120°C durchgeführt wird.

## Claims

1. Process for the preparation of 4-substituted 3-methyl-1-aryl-5-amino-pyrazoles of the general formula (I) 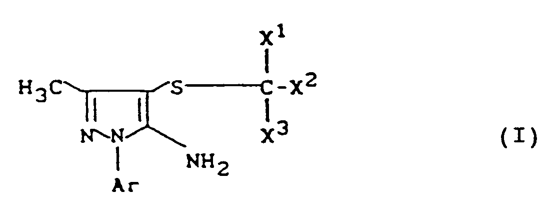 in which
X¹, X² and X³ independently of one another each stand for fluorine, chlorine or bromine and
Ar stands for phenyl which is optionally monosubstituted to pentasubstituted by identical or different substituents, suitable substituents in each case being: cyano, nitro, fluorine, chlorine, bromine, iodine, methyl, ethyl, n- or i-propyl, n-, i-, s-, or t-butyl, methoxy, ethoxy, methoxycarbonyl, ethoxycarbonyl, trifluoromethyl, trichloromethyl, dichlorofluoromethyl, difluorochloromethyl, chloromethyl, dichloromethyl, difluoromethyl, pentafluoroethyl, tetrafluoroethyl, trifluorochloroethyl, trifluoroethyl, difluorodichloroethyl, trifluorodi-chloroethyl, pentachloroethyl, trifluoromethoxy, trichloromethoxy, dichlorofluoromethoxy, difluorochloromethoxy, chloromethoxy, dichloromethoxy, difluoromethoxy, pentafluoroethoxy, tetrafluoroethoxy, trifluorochloroethoxy, trifluoroethoxy, difluorodichloroethoxy, trifluorodichloroethoxy, pentachloroethoxy or an -S(O)ₙ-R³ radical, where
R³ stands for amino, methylamino, ethylamino, dimethylamino, diethylamino, fluorodichloromethyl, difluoromethyl, tetrafluoroethyl, trifluorochloroethyl, trichloromethyl, trichloroethyl, trifluoromethyl, methyl or ethyl, and
n stands for a number 0, 1 or 2,
characterized in that 3-ketobutyraldehyde dimethyl acetal of the formula (II) 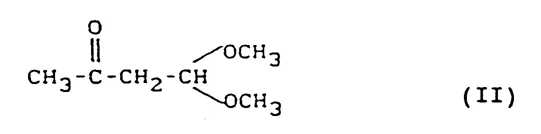 is first reacted with amines of the formula (III) 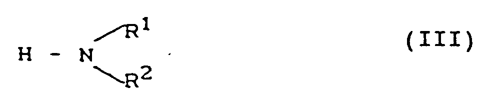 in which
R¹ and R³ independently of one another each stand for hydrogen, alkyl, cycloalkyl or for in each case optionally substituted aralkyl, aryl or heteroaryl, or together with the nitrogen atom to which they are bonded stand for an optionally substituted heterocyclic ring which can optionally contain further hetero atoms,
if appropriate in the presence of a diluent, the reaction mixture is then further reacted directly without isolation of the intermediates of the formula (IV) 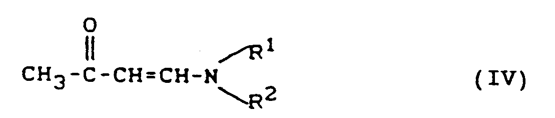 in which
R¹ and R² have the abovementioned meaning,
with trihalogenomethanesulphenyl chlorides of the formula (V) 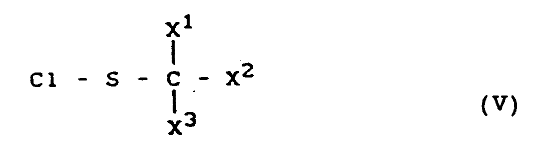 in which
X¹, X² and X³ have the abovementioned meaning,
if appropriate in the presence of a diluent and if appropriate in the presence of an acid-binding agent, the reaction mixtures are then further reacted directly, without isolation of the intermediates of the formula (VI) 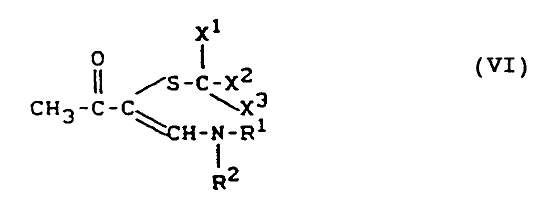 in which
R¹, R², X¹, X² and X³ have the abovementioned meaning, with hydroxylamine hydrochloride in the presence of an acid-binding agent and if appropriate in the presence of a diluent, and the reaction mixture is then further reacted directly with arylhydrazines of the formula (IX)
Ar-NH-NH₂ (IX)
in which
Ar has the abovementioned meaning,
without isolation of the intermediates of the formula (VII) 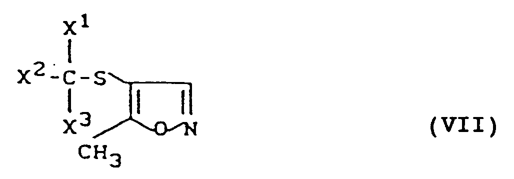 which, under the given reaction conditions, immediately react further to give the open-chain tautomers of the formula (VIIIa) 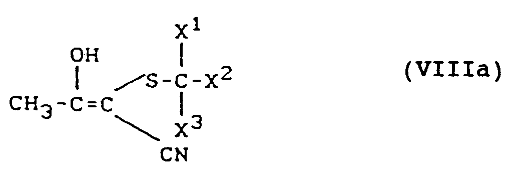 in which
X¹, X² and X³ have the abovementioned meaning,
and of the formula (VIIIb) 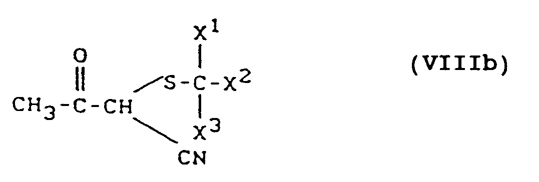 in which
X¹, X² and X³ have the abovementioned meaning,
if appropriate in the presence of a diluent and in the presence of an acid reaction auxiliary.

2. Process according to Claim 1 for the preparation of compounds of the formula (I) in which
X¹, X² and X³ independently of one another each stand for fluorine or chlorine and
Ar stands for phenyl which is optionally monosubstituted to pentasubstituted by identical or different substituents, suitable substituents being: fluorine, chlorine, bromine, methyl, ethyl, methoxy, ethoxy, trifluoromethyl, trifluoromethoxy, trifluoromethylthio, trifluoromethylsulphinyl or trifluoromethylsulphonyl.

3. Process according to Claim 1 or 2, characterized in that inert organic diluents or mixtures thereof with water or water on its own are employed as the diluent.

4. Process according to Claim 1 to 3, characterized in that aliphatic, alicyclic or aromatic optionally halogenated hydrocarbons, ethers, amides, esters, sulphoxides, alcohols, mixtures thereof with water or water are employed as the diluent.

5. Process according to Claim 1 to 4, characterized in that water or water/alcohol mixtures are employed as the diluent.

6. Process according to Claim 1 to 5, characterized in that the process is carried out in the presence of an inorganic or organic base as the acid-binding agent.

7. Process according to Claim 1 to 5, characterized in that the final reaction step is carried out in the presence of an acidic reaction auxiliary from the series comprising inorganic mineral acids, such as hydrochloric acid or sulphuric acid; aliphatic or aromatic carboxylic or sulphonic acids, such as acetic acid, methanesulphonic acid or p-toluenesulphonic acid; Lewis acids, such as boron trifluoride, iron trichloride, aluminium trichloride or zinc dichloride or acid ion exchangers.

8. Process according to Claim 1 to 7, characterized in that the reaction is carried out at temperatures of between 0°C and +120°C.

## Revendications

1. Procédé de préparation de 3-méthyl-1-aryl-5-aminopyrazoles substitués en position 4 de formule générale (I) 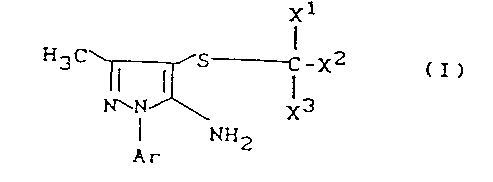 dans laquelle
X¹, X² et X³ représentent chacun indépendamment l'un de l'autre le fluor, le chlore ou le brome et
Ar représente un phényle ayant éventuellement un à cinq substituants identiques ou différents, les substituants entrant en ligne de compte étant à chaque fois les substituants cyano, nitro, fluor, chlore, brome, iode, méthyle, éthyle, n- ou i-propyle, n-, i-, s- ou t-butyle, méthoxy, éthoxy, méthoxycarbonyle, éthoxycarbonyle, trifluorométhyle, trichlorométhyle, dichlorofluorométhyle, difluorochlorométhyle, chlorométhyle, dichlorométhyle, difluorométhyle, pentafluoroéthyle, tétrafluoroéthyle, trifluorochloroéthyle, trifluoroéthyle, difluorodichloroéthyle, trifluorodichloroéthyle, pentachloroéthyle, trifluorométhoxy, trichlorométhoxy, dichlorofluorométhoxy, difluorochlorométhoxy, chlorométhoxy, dichlorométhoxy, difluorométhoxy, pentafluoroéthoxy, tétrafluoroéthoxy, trifluorochloroéthoxy, trifluoroéthoxy, difluorodichloroéthoxy, trifluorodichloroéthoxy, pentachloroéthoxy ou un reste -S(O)ₙ-R³ où
R³ représente un reste amino, méthylamino, éthylamino, diméthylamino, diéthylamino, fluorodichlorométhyle, difluorométhyle, tétrafluoroéthyle, trifluorochloroéthyle, trichlorométhyle, trichloroéthyle, trifluorométhyle, méthyle ou éthyle, et
n représente le chiffre 0, 1 ou 2,
caractérisé en ce qu'on fait réagir du diméthylacétal de 3-oxobutyraldéhyde de formule (II) 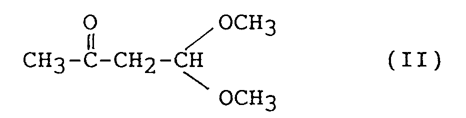 d'abord avec des amines de formule (III) 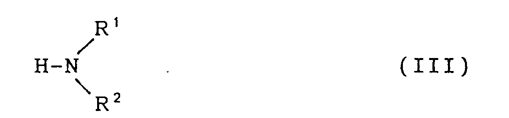 dans laquelle R¹ et R² sont chacun indépendamment l'un de l'autre l'hydrogène, un alkyle, un cycloalkyle ou un aralkyle, aryle ou hétéroaryle pouvant chacun être substitué, ou bien ils forment ensemble, avec l'atome d'azote auquel ils sont liés, un hétérocycle éventuellement substitué qui peut contenir éventuellement d'autres hétéroatomes,
éventuellement en présence d'un diluant, puis on fait réagir directement plus avant le mélange réactionnel, sans isoler les produits intermédiaires de formule (IV), 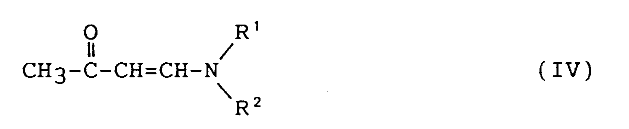 dans laquelle R¹ et R² ont la signification donnée ci-dessus,
avec des chlorures de trihalogénométhanesulfényle de formule (V) 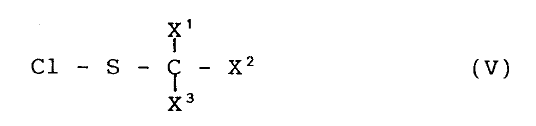 dans laquelle X¹, X² et X³ ont la signification donnée ci-dessus,
éventuellement en présence d'un diluant et éventuellement en présence d'un agent fixateur d'acide, puis on fait réagir directement plus avant le mélange réactionnel, sans isoler les produits intermédiaires de formule (VI) 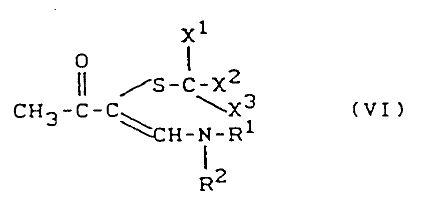 dans laquelle R¹, R², X¹, X² et X³ ont la signification donnée ci-dessus,
avec du chlorhydrate d'hydroxylamine en présence d'un agent fixateur d'acide et éventuellement en présence d'un diluant, puis on fait réagir directement plus avant le mélange réactionnel, sans isoler les produits intermédiaires de formule (VII) 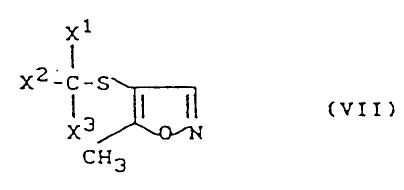 qui continuent aussitôt à réagir dans les conditions données de la réaction pour former les tautomères à chaîne ouverte de formule (VIIIa) 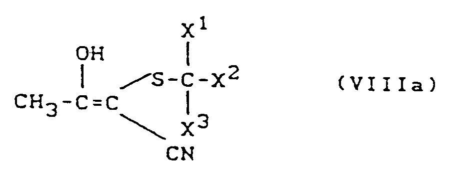 dans laquelle X¹, X² et X³ ont la signification donnée ci-dessus,
et de formule (VIIIb) 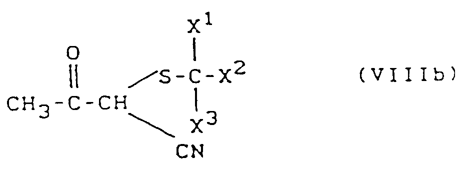 dans laquelle X¹, X² et X³ ont la signification donnée ci-dessus,
avec des arylhydrazines de formule (IX)
Ar - NH - NH₂ (IX)
dans laquelle Ar a la signification donnée ci-dessus,
éventuellement en présence d'un diluant et en présence d'un auxiliaire de réaction acide.

2. Procédé selon la revendication 1 pour la préparation de composés de formule (I) dans laquelle
X¹, X² et X³ sont chacun indépendamment l'un de l'autre le fluor ou le chlore et
Ar représente un phényle qui peut éventuellement porter un à cinq substituants identiques ou différents, les substituants entrant en ligne de compte étant les substituants fluor, chlore, brome, méthyle, éthyle, méthoxy, éthoxy, trifluorométhyle, trifluorométhoxy, trifluorométhylthio, trifluorométhylsulfinyle ou trifluorométhylsulfonyle.

3. Procédé selon les revendications 1 et 2, caractérisé en ce qu on utilise comme diluants des diluants organiques inertes ou leurs mélanges avec de l'eau, ou de l'eau seule.

4. Procédé selon les revendications 1 à 3, caractérisé en ce qu'on utilise comme diluants des hydrocarbures aliphatiques, alicycliques ou aromatiques, éventuellement halogénés, des éthers, des amides, des esters, des sulfoxydes, des alcools, leurs mélanges avec de l'eau, ou bien de l'eau.

5. Procédé selon les revendications 1 à 4, caractérisé en ce qu'on utilise comme diluants de l'eau ou des mélanges eau-alcool.

6. Procédé selon les revendications 1 à 5, caractérisé en ce qu'on travaille en présence d'une base inorganique ou organique comme agent fixateur d'acide.

7. Procédé selon les revendications 1 à 5, caractérisé en ce que, dans la dernière étape réactionnelle, on travaille en présence d'un auxiliaire de réaction acide de la série des acides minéraux inorganiques, tels que l'acide chlorhydrique ou l'acide sulfurique; des acides carboxyliques ou sulfoniques aliphatiques ou aromatiques, tels que l'acide acétique, l'acide méthanesulfonique ou l'acide p-toluènesulfonique; des acides de Lewis, tels que le trifluorure de bore, le trichlorure de fer, le trichlorure d'aluminium ou le dichlorure de zinc, ou des échangeurs d'ions acides.

8. Procédé selon les revendications 1 à 7, caractérisé en ce que la réaction est effectuée à des températures comprises entre 0°C et +120°C.
